# EUROPEAN PATENT SPECIFICATION

(11) **EP 4 176 925 B1**
(45) Date of publication and mention of the grant of the patent: **18.03.2026**
(21) Application number: 22205902.4
(22) Date of filing: 07.11.2022
(51) Int. Cl.: A61N 1/375, A61N 1/378, A61N 1/372

(54) **LEADLESS ACTIVE IMPLANTABLE MEDICAL DEVICE HAVING ELECTRODES CO-FIRED ONTO ITS CERAMIC HOUSING**
LEITUNGSLOSE AKTIVE IMPLANTIERBARE MEDIZINISCHE VORRICHTUNG MIT AUF SEINEM KERAMISCHEN GEHÄUSE EINGEBRANNTEN ELEKTRODEN
DISPOSITIF MÉDICAL IMPLANTABLE ACTIF SANS FIL AYANT DES ÉLECTRODES CO-CUITES SUR SON BOÎTIER CÉRAMIQUE

(30) Priority: 05.11.2021 US 202163276031 P
(43) Date of publication of application: 10.05.2023
(73) Proprietor: Greatbatch Ltd., Clarence, New York 14031 (US)
(72) Inventor: VILLAMIL, Luis, CP 11400 Montevideo (UY); ARMESTO, Ignacio, CP 11600 Montevideo (UY); FERNÁNDEZ, Federica, CP 11200 Montevideo (UY)
(74) Representative: Mathys & Squire

(56) References cited:
- WO-A1-2021/008688
- US-A1- 2011 288 615
- US-A1- 2012 303 105
- US-A1- 2017 303 424
- US-B1- 8 805 537

## Description

### FIELD OF THE INVENTION

The present invention relates to the field of implantable medical devices. More particularly, the present invention relates to a leadless active implantable medical device (AIMD) that is designed to deliver electrical stimulation to a patient or sense biological signals from body tissue.

The leadless AIMD of the present invention may have an elongate ceramic housing. At least two spaced-apart superficial electrodes may be supported on an outer surface of the ceramic housing. The electrodes can be ring-shaped to electrically stimulate body tissue or sense biological signals in a 360° field about the ceramic housing, or they can be discrete electrodes to provide current to body tissue and/or to sense electrical signals from body tissue in a radiating pattern that is substantially directly in front of them. As an inherently electrically insulative material, the ceramic material forming the housing electrically isolates the electrodes from each other. A charging coil housed inside the housing may be configured to charge a Li-ion battery or capacitor that is connected to a printed circuit board (PCB) assembly contained inside the housing. The PCB assembly may comprise a PCB supporting a plurality of electronic components that control the various functions of the medical device, including having the electrodes deliver electrical stimulation to a patient and sense biological signals from body tissue. The electrodes may be connected to the PCB assembly by electrically conductive pathways comprising a via extending through the housing and containing a platinum-containing paste that is co-sintered with the housing when in a green-state.

### BACKGROUND OF THE INVENTION

Conventional leadless implantable medical devices have a metallic housing. The metallic housing supports at least two electrodes that are configured to send electrical pulses to the surrounding body tissue or sense biological signals from the body tissue. The stimulation and sensing electrodes are electrically energized by electronic circuits housed inside the medical device. Since the stimulation and sensing electrodes need to be in contact with the surrounding body tissue, they must be hermetically sealed in the device housing or its header and electrically isolated from the metallic housing. In that respect, including the features needed to support and hermetically seal them in the device housing, the stimulation and sensing electrodes and their associated support structures consume a relatively large amount of space in the medical device.

Moreover, the metallic housing can interfere with two-way communication between the medical device and an associated patient programmer or clinician programmer. These programmers are used by the patient and the clinician to configure the medical device to operate in a desired manner. A patient programmer is used by the patient in whom the medical device is implanted to adjust the parameters of electrical stimulation delivered by the device. A clinician programmer is used by medical personnel to configure and adjust stimulation parameters that the patient is not permitted to control. However, a metallic device housing may interfere with the inductive signals that are transmitted back and forth from the patient and clinician programmers to the medical device. A metallic device housing may also interfere with the inductive or RF charging signals that are used to recharge the electrical power source of the medical device.

US2017/303424 A1 shows an implantable medical device comprising a hybrid circuitry assembly and a core circuitry support structure comprising a frame defining a cavity configured to receive at least a portion of the hybrid circuitry assembly, wherein an outer surface of the frame is shaped to correspond to an inside surface of a core assembly housing configured to enclose the hybrid circuitry assembly and the core circuitry support structure.

Further implantable medical devices are known from US 2011/288615 A1, US 8 805 537 B1 and US 2012/303105 A1.

To overcome these shortcomings, the communication/charging antenna, whether there are two dedicated antennas or an integrated antenna, must be placed outside the metallic housing, typically inside a polymeric header connected to the device housing. Positioning the communication/charging antenna in the device header requires a significant amount of space that, if eliminated, could greatly reduce the size of the implantable medical device. A smaller device is easier to implant in a patient and would be expected to cause less trauma to the patient.

Therefore, there is a desire to minimize the space occupied by the stimulation and sensing electrodes in a leadless active implantable medical device.

### SUMMARY OF THE INVENTION

The present invention is defined by the appended claims.

Aspects and embodiments described hereinafter are of exemplary nature and presented for better understanding the present invention which is defined by the appended claims.

The purpose of the present invention is to reduce the size of a leadless implantable medical device by reducing the volume of the electrodes, reducing or eliminating the features needed to hermetically seal the electrodes to the housing, reducing the number of components or processes needed to insulate the electrodes from each other and from the other electrical components of the medical device, and/or reducing the volume of the connections between the electrodes and the electrical components supported on a printed circuit board contained inside the medical device housing.

The leadless implantable medical device of the present disclosure has a housing that may be made from a ceramic material. At least two superficial stimulation or sensing electrodes may be supported on the ceramic housing, spaced-apart from each other. Since the electrodes are superficial structures, lying on the outer surface of the device housing, they occupy very little space. And, since the ceramic forming the device housing is an inherently electrically insulative material, the at least two electrodes supported on the housing are electrically isolated from each other and from the rest of the device components without any additional support or isolation structure.

Respective electrically conductive pathways may connect from the electrodes to electronic circuits supported on a printed circuit board (PCB) contained inside the ceramic device housing. The conductive pathways may be formed from an electrically conductive paste, preferably a platinum-containing paste, that is filled into vias aligned with a respective one of the electrodes and extending through the ceramic housing. With the ceramic forming the housing being in a green-state, the housing supporting the superficial electrodes and the conductive paste in the housing vias may be co-fired in a sintering operation. Sintering solidifies the ceramic housing into the desired shape for the medical device, bonds the superficial electrodes to the ceramic housing and simultaneously transforms the conductive paste in the vias into solid, electrically conductive pathways. An outer end of each conductive pathway may contact an inner surface of a respective one of the electrodes while an inner end of the conductive pathway may be in electrical continuity with an electrical contact supported on the PCB. The electrical contacts connect to electronic circuits of the PCB, and in turn, the electronic circuits draw power from an electrical power source connected to the device housing. In that manner, the at least two spaced-apart superficial electrodes may be energized to either provide electrical stimulation to body tissue in which the medical device is implanted, sense biological signals from adjacent body tissue, or both. Thus, providing the at least two spaced-apart electrodes as superficial electrodes supported on the ceramic housing means that the amount of space the electrodes and their connections to the electrical components supported on the PCB represents a significant improvement in space savings in comparison to a convention leadless implantable medical device having a metallic housing.

Moreover, the ceramic housing does not interfere with the communication/charging fields that are transmitted between external devices and the medical device when it is implanted in body tissue. These include communication signals from patient and clinician programmers to the implanted medical device, and inductive or RF (radio frequency) charging signals sent from an external charging transmitter to the power source charging antenna. This means that the communication and charging antennas can be placed inside the ceramic housing, which further reduces the device's volume and manufacturing complexity.

Furthermore, the spaced-apart electrodes may be provided as ring-shaped electrodes which means that the leadless implantable medical device can contact surrounding body tissue in a 360° geometry about the medical device. However, the present disclosure is not limited to a defined number of electrodes; there are at least two electrodes in a system, and they can be at least one ring-shaped electrode combined with at least one discrete electrode that is not ring-shaped.

These and other aspects of the present disclosure will become increasingly more apparent to those skilled in the art by reference to the following detailed description and to the appended drawings.

### BRIEF DESCRIPTION OF THE DRAWINGS

FIG. 1 is a simplified block diagram of an exemplary medical system 10.
FIG. 2 is a perspective view of an exemplary embodiment of the implantable medical device 12 shown in the medical system 10 illustrated in FIG. 1.
FIG. 3 is a side elevation view of the implantable medical device 12 shown in FIG. 2.
FIG. 4 is a cross-sectional view taken along line 4-4 of FIG. 3.
FIG. 5 is an enlarged view of the indicated area in FIG. 4.
FIG. 6 is a partially exploded view of the implantable medical device 12 shown in FIG. 2 looking at the charging coil 50 supported on the printed circuit board (PCB) 48.
FIG. 7 is a plan view of the implantable medical device 12 shown in FIG. 2.
FIG. 8 is a cross-sectional view taken along line 8-8 of FIG. 7.
FIG. 9 is an enlarged view of the indicated area in FIG. 8.
FIG. 10 is a partially exploded view of the implantable medical device 12 shown in FIG. 2 looking at the bottom side of the printed circuit board (PCB) 48, opposite the charging coil 50.
FIG. 11 is a perspective view of another exemplary embodiment of an implantable medical device 100 that is useful in the medical system 10 illustrated in FIG. 1.
FIG. 12 is a side elevational view of the implantable medical device 100 shown in FIG. 11.
FIG. 13 is a plan view of the implantable medical device 100 shown in FIG. 11.
FIG. 14 is a cross-sectional view taken along line 14-14 of FIG. 13.

### DETAILED DESCRIPTION OF THE PPRESENT DISCLOSURE

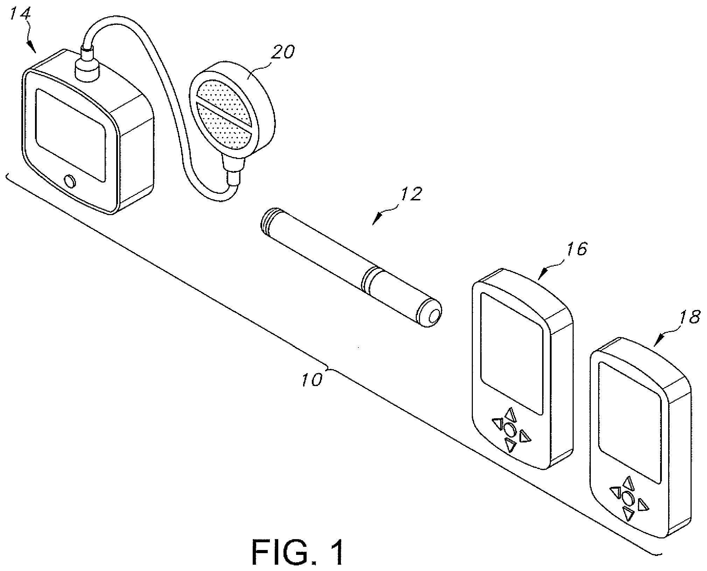

Turning now to the drawings, FIG. 1, illustrates a simplified block diagram of an exemplary medical system 10. The medical system 10 includes a leadless active implantable medical device (AIMD) 12, which can be any one of various types of known implantable medical devices that can be implanted in a patient's body tissue. The medical system 10 also has an external charger 14, a patient programmer 16, and a clinician programmer 18.

The patient programmer 16 and the clinician programmer 18 may be portable handheld devices, such as a smartphone or other custom device, that are used to configure the AIMD 12 so that the AIMD can operate in a desired manner. The patient programmer 16 is used by the patient in whom the AIMD 12 is implanted. The patient may adjust the parameters of electrical stimulation delivered by the AIMD 12, such as by selecting a stimulation program, changing the amplitude and frequency of the electrical stimulation, among other parameters, and by turning stimulation on and off.

The clinician programmer 18 is used by medical personnel to configure the other system components and to adjust stimulation parameters that the patient is not permitted to control. These include setting up stimulation programs among which the patient may choose and setting upper and lower limits for the patient's adjustments of amplitude, frequency, and other parameters. It is also understood that although FIG. 1 illustrates the patient programmer 16 and the clinician programmer 18 as two separate devices, they may be integrated into a single programmer in some embodiments.

Electrical power may be delivered to the AIMD 12 through an external charging pad 20 that is connected to the external charger 14. In some embodiments, the external charging pad 20 is configured to directly power the AIMD 12 or it is configured to charge a rechargeable electrical energy power source 24 of the AIMD. The external charging pad 20 can be a hand-held device that is connected to the external charger 14, or it can be an internal component of the external charger 14.

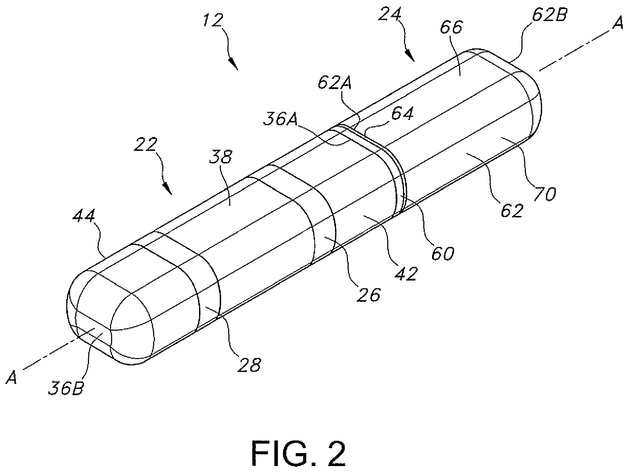

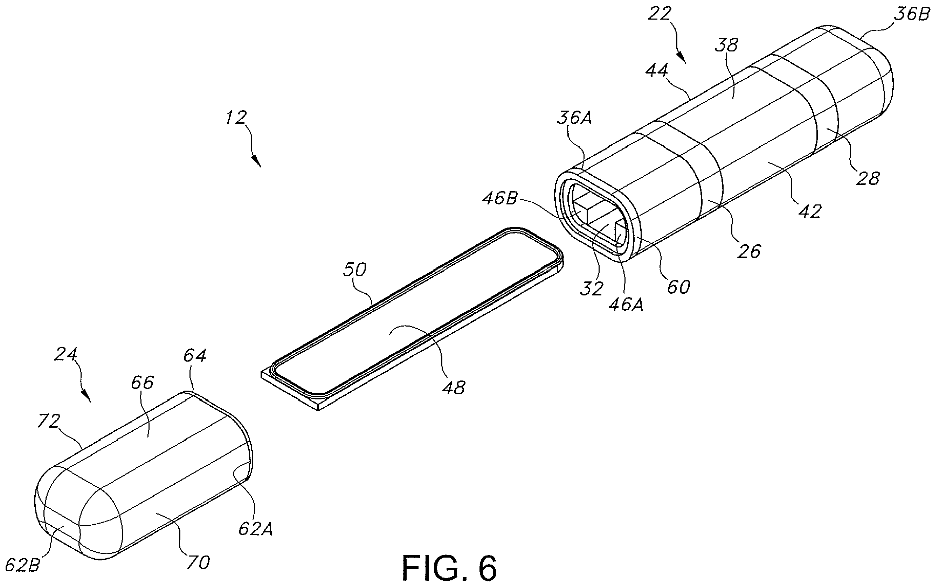

Referring now to FIGs. 2 to 7, an exemplary embodiment of the leadless active implantable medical device (AIMD) 12 shown in FIG. 1 is illustrated. The AIMD 12 is an elongate device that extends longitudinally along an axis A-A with an exemplary length of about 33 mm and a diameter of about 4 mm. However, the shape of the AIMD 12 is not limited to the elongate shape that is shown. For example, the AIMD 12 could have a cylindrical shape or a shape that is not elongated.

The AIMD 12 comprises a non-conductive, elongate housing 22, for example, of a ceramic material, connected to an electrical energy power source 24. However, the material of the device housing 22 is not limited to ceramic materials; other non-conductive materials, for example, glass (e.g., HPFS) or plastic (e.g., PEEK) may be used as long as they are biocompatible and offer the appropriate mechanical robustness.

The electrical energy power source 24 can be a capacitor or a rechargeable battery, for example a hermetically sealed rechargeable Li-ion battery. However, the electrical energy power source 24 is not limited to any one chemistry or even a rechargeable chemistry and can be of an alkaline cell, a primary lithium cell, a rechargeable lithium-ion cell, a Ni/cadmium cell, a Ni/metal hydride cell, a supercapacitor, a thin film solid-state cell, and the like. Preferably, the electrical energy power source 24 is a lithium-ion electrochemical cell comprising a carbon-based or Li₄Ti₅O₁₂-based anode and a lithium metal oxide-based cathode, such as of LiCoO₂ or lithium nickel manganese cobalt oxide (LiNiₐMn_{b}Co_{1-a-b}O₂). The electrical energy power source 24 can also be a solid-state thin film electrochemical cell having a lithium anode, a metal-oxide based cathode and a solid electrolyte, such as an electrolyte of LiPON (LiₓPO_{y}N_{z}).

The AIMD 12 supports at least two spaced-apart bandshaped or ring-shaped electrodes 26 and 28. The ring-shaped electrodes 26 and 28 are configured to provide current to body tissue and/or to sense electrical signals from body tissue. While two electrodes 26, 28 are shown in the exemplary embodiment of the present implantable medical device 12, that is the minimum number of electrodes that is needed for a functioning medical device. It is contemplated that there can three, four, and possibly more electrodes comprising the AIMD 12.

In more detail, the device housing 22 is formed from a ceramic material that has a relatively high dielectric constant. An essentially pure alumina is a preferred ceramic material. The term "essentially pure alumina" refers to an alumina ceramic having the chemical formula Al₂O₃. "Essentially pure" means that the post-sintered alumina is at least 96% alumina. In a preferred embodiment, the post-sintered alumina is at least 99% high purity alumina.

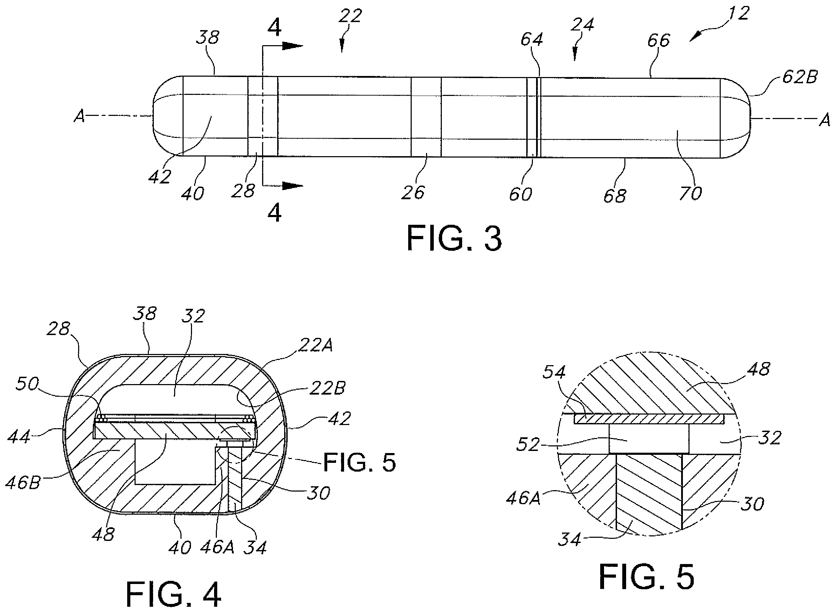

Prior to sintering, the alumina comprising the device housing 22 may be a paste, a slurry, or a green-state ceramic that is injection molded, powder pressed, and the like, into the desired shape of the housing as a single monolithic structure. The green-state alumina ceramic is very pliable due to the organic binders and solvents that have been temporarily added to the system. It is at this step that at least two vias 30 (only one via is shown in FIGs. 4 and 5) are formed through the green-state ceramic from an outer or body fluid side surface 22A of the housing 22 to an inner or device side surface 22B (electronics side) defining an interior space 32 inside the alumina housing 22. Drilling is a preferred method for forming the vias 30, but they may also be formed by punching, laser drilling, water cutting, or any other equivalent process. The at least two vias 30 are aligned with a respective one of the at least two electrodes 26 and 28 that will subsequently be supported on the green-state ceramic comprising the device housing 22.

After the vias 30 are formed, a pure platinum paste composition is injected under pressure or via vacuum into the vias. The pressure or vacuum is carefully controlled to drive the platinum paste into intimate contact with the ceramic surface surrounding the vias so that the paste conforms to and creates a mirror image of the inner surface of the ceramic material defining the vias 30 and, in so doing, the platinum paste interconnects with the already tortuous members prevalent in ceramic materials.

The thermal expansion of metals, for example platinum, is generally considerably greater than that of ceramics, for example alumina. For example, at a bakeout temperature of about 500°C., the CTE of alumina is 7.8x10⁻⁶/K and of platinum is 9.6x10⁻⁶/K. Historically, CTE differences within a range of from about 0.5 x10⁻⁶/K to about 1.0x10⁻⁶/K between the mating metal and ceramic materials are adequate to sustain hermetic bonding between these materials. Hence, a hermetic seal is formed between the sintered platinum 34 in the vias 30 and the ceramic material comprising the device housing 22 through the controlled fabrication process parameters of the platinum metal particle solids loading within the paste, controlled packing of the platinum paste within the vias 30, and the controlled shrinkage of the alumina device housing 22 and platinum via paste through a prescribed co-sintering process.

In that respect, a highly irregular surface at the material interface between the alumina housing 22 and the platinum metal particles within the vias 30 provides a mechanical contribution to adherence and robustness of the hermetic seal between the alumina and the platinum 34. A surface roughness produced by drill bits, sandblasting, grit blasting or chemical etching of the vias 30 through the device housing 22 helps to increase the surface area at the vias and, in so doing, provides for a stronger mechanical attachment along the mutually conformal interface. Examples of sandblasting and grit blasting media include sand, sodium bicarbonate, walnut shells, alumina particles or other equivalent media.

Thus, to achieve sustainable hermeticity between the alumina housing 22 and the platinum metal particles 34 within the vias 30, the following is required. Because the CTE of platinum (9.6x10⁻⁶/K) is sufficiently higher than the CTE of alumina (7.8x10⁻⁶/K), the platinum material 34 filled into the ceramic vias 30 must be formed using a paste, a slurry, or the like, having a minimum of about 80% solids loading. A paste is defined as a flowable medium having a viscosity that ranges in centipoise (cP) from about 1 x 10⁵ cP to about 1 x 10¹⁰ cP.

In a preferred embodiment, the solids loading of the platinum particles within the paste is about 90%. In a more preferred embodiment, the solids loading of the platinum particles within the paste is about 95%. In addition, the vias 30 must be packed with the platinum paste to occupy at least 90% of the available space within each via. In a preferred embodiment, the platinum paste is packed within the via to occupy about 95% of the open space. In a more preferred embodiment, the platinum paste is packed to occupy about 99% of the via opening. The shrinkage of the alumina must be no greater than about 20% of that of the platinum paste filled into the vias 30. In a preferred embodiment, shrinkage is about 14%. In a more preferred embodiment, shrinkage is about 16%.

Referring now to the at least two spaced-apart bandshaped or ring-shaped electrodes 26 and 28, they are of an electrically conductive material, preferably platinum or a platinum alloy, supported in matching shaped recesses in the body fluid side surface 22A of the green-state ceramic housing 22. The electrodes 26 and 28 are aligned and in direct contact with the body fluid ends of the platinum paste filled into a respective one of the at least two vias 30. Since the electrodes 26 and 28 will be exposed to body fluids, and the like, they must be of a biocompatible material. In addition to platinum and its alloys, suitable biocompatible materials include gold, gold alloys, rhodium, titanium, molybdenum, and mixtures thereof. The electrodes 26 and 28 may be applied to the outer or body fluid side surface 22A of the green-state ceramic housing 22 by thin and thick film technologies, such as printing, screen printing, pad printing, painting, plating, brush coating, direct bonding, active metal brazing, magnetron sputtering, physical vapor deposition, ion implantation, electroplating, and electroless plating.

As an assembly, the green-state ceramic housing 22 supporting the ring-shaped electrodes 26, 28 aligned and in direct contact with the platinum paste filled vias 30 is then subjected to a controlled co-firing or co-sintering process in ambient air that comprises a binder bakeout portion, a sintering portion, and a cool down portion. The binder bakeout portion is performed at a temperature that ranges from about 400°C. to about 700°C. for a minimum of about 4 hours. A preferred binder bakeout is at a temperature that ranges from about 550°C. to about 650°C. A more preferred binder bakeout is at a temperature that ranges from about 500°C. to about 600°C.

The sintering portion is preferably performed at a temperature that ranges from about 1,400°C. to about 1,900°C. for up to about 6 hours. A preferred sintering profile has a temperature that ranges from about 1,500°C. to about 1,800°C. A more preferred sintering temperature ranges from about 1,600°C. to about 1,700°C.

The cool down portion occurs either by turning off the heating chamber and allowing the chamber to equalize to room temperature or, preferably by setting the cool down portion at a rate of up to about 5°C./min from the hold temperature cooled down to about 1,000°C. At about 1,000°C., the chamber is allowed to naturally equalize to room temperature. A more preferred cool down is at a rate of about 1°C./min from the hold temperature to about 1,000°C. and then the heating chamber is allowed to naturally equalize to room temperature. In so doing, the desired outcome of achieving robust electrical connections of the platinum 34 forming a conductive pathway connecting from each of the electrodes 26 and 28 to the interior space 32 inside the device housing and the hermetic seal formed between the mating materials of the alumina device housing 22 and the platinum 34 hermetically sealed to the ceramic material of the housing in the vias 30 is achieved.

Once the binders and solvents have been driven out of the system and sintering has occurred, the result is a solid monolithic high purity alumina device housing 22 supporting the at least two electrodes 26 and 28 aligned and in direct physical and electrical contact with a body fluid side end of a respective one of the at least two conductive pathways 34 in the vias 30. The sintering process has transformed the platinum paste in the vias 30 into solid electrically conductive pathways 34 comprising platinum extending from the outer or body fluid side surface 22A of the housing where they directly electrically and physically contact the electrodes 26 and 28 to the inner surface 22B thereof defining the interior space 32 inside the device housing. A mutually conformal hermetic interface is thereby formed between the platinum conductive pathways 34 and the ceramic material of the device housing 22 defining the vias 30.

The sintered high purity alumina device housing 22 has a surrounding sidewall extending from a proximal annular rim 36A to a closed distal end 36B. The proximal rim 36A surrounds and defines an opening leading into the interior space 32 inside the housing 22. In that respect, the device housing 22 is a hollow member that is comprised of opposed upper and lower face walls 38 and 40 that extend to and meet with opposed curved edge walls 42 and 44. Together, the face walls 38, 40 and edge walls 42, 44 extend from the proximal rim 36A to the curved closed distal end 36B.

As shown in FIGs. 4, 6 and 7, a pair of integral side-byside rails 46A and 46B resides inside the interior space 32 inside the device housing 22. The integral rails 46A, 46B are aligned along, but spaced on opposite side of, the longitudinal axis A-A and extend from adjacent to the proximal rim 36A to the closed distal end 36B.

A printed circuit board (PCB) assembly 48 resides in the interior space 32 inside the device housing 22, supported on the rails 46A, 46B. The PCB 48 supports at least one, and preferably a plurality of electronic components (not shown) as an assembly that controls the various functions performed by the AIMD 12. These include, but are not limited to, receiving sensed electrical signals pertaining to functions of the body tissue in which the AIMD 12 is implanted and for delivering electrical current pulses to the body tissue through the electrodes 26 and 28. The PCB 48 also supports a charging coil 50 connected to a charging circuit (not shown). The charging circuit is configured to convert RF or inductive energy signals received by the inductive charging coil 50 from the external charging pad 20 connected to the external charger 14 (FIG. 1) into a direct current voltage to charge the electrical power source 24 to power the electronic components of the PCB 48.

As shown in FIGs. 4 and 5, with the PCB 48 residing in the interior space 32, supported on the rails 46A, 46B, inside the device housing 22, an electrical connection is made from the electrically conductive pathways 34 at the inner surface 22B of the device housing to the PCB by a re-flowed solder 52. There are two solders 52, each contacting one of the conductive pathways 34 and an electrical contact 54 supported on the PCB 48. The electrical contact 54 is in electrical continuity with the electronic components supported on the PCB 48.

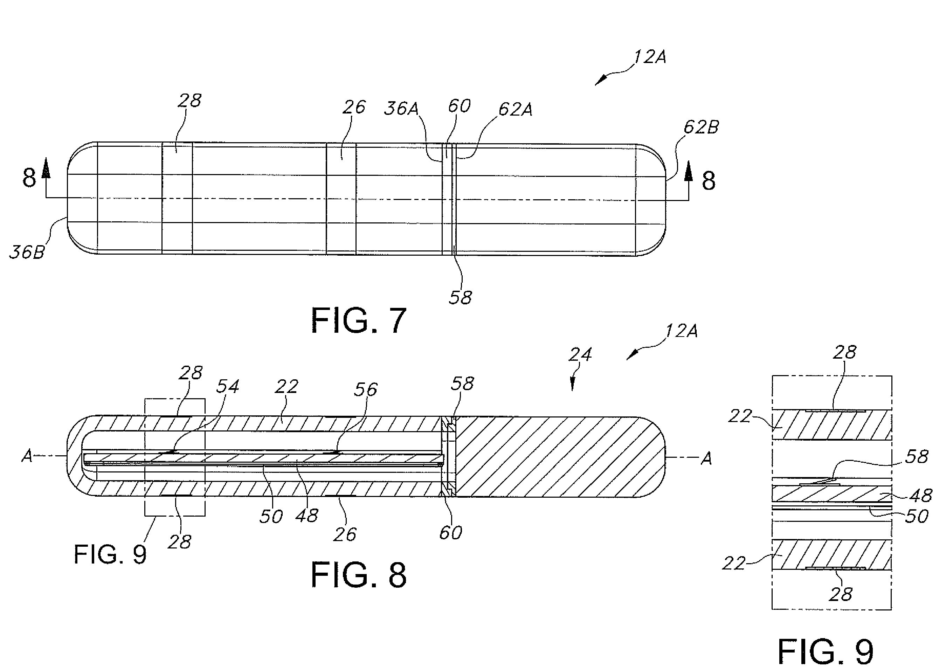

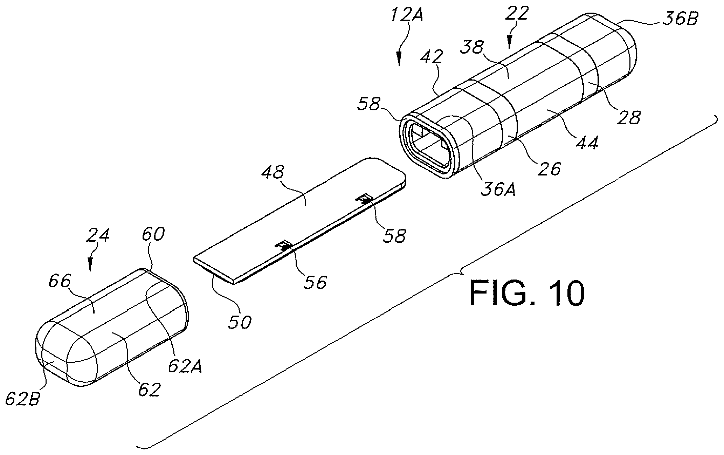

FIGs. 7 to 10 illustrate an alternate embodiment for electrically connecting the electronic components supported on the PCB 48 to the electrodes 26, 28. In this embodiment, the PCB 48 supports the same number of leaf springs as there are electrodes. Since the illustrated embodiment shows two electrodes 26, 28, there are two leaf springs 56 and 58 corresponding to a respective one of the electrodes. The leaf springs 56 and 58 are spaced apart and configured to bias toward the PCB as the PCB 48 is moved along the rails 46A, 46B into the interior space 32 inside the device housing 22. Once the PCB is properly positioned inside the device housing 22, the leaf springs 56 and 58 bias into direct physical and electrical contact with the conductive pathways 34 in turn electrically contacting the electrodes 26 and 28.

As shown in FIGs. 2, 3, 6 to 8, an annular flange 60 of a biocompatible material, for example titanium, is supported at the proximal rim 36A of the device housing. To connect the ceramic housing 22 to the flange 60, the outwardly facing edge of the rim 36A is provided with a metallization (not shown). A suitable metallization comprises two metallization layers, a first adhesion layer that is directly applied to the outwardly facing edge of the rim 36A, and a second, wetting layer, which is applied on top of the adhesion layer. In a preferred embodiment, the adhesion layer is titanium, and the wetting layer is either molybdenum or niobium.

The adhesion and wetting metallization layers may be applied to the device housing rim 36A by thin and thick film technologies, such as printing, painting, plating, and deposition processes. Metallization processes include screen printing, pad printing, brush coating, direct bonding, active metal brazing, magnetron sputtering, physical vapor deposition, ion implantation, electroplating, and electroless plating. In an alternate embodiment, both the adhesion and wetting metallization layers may be provided by a single metallization layer. It is noted that in the present drawings, the adhesion and wetting layers are intentionally not shown for the sake of simplicity.

A braze pre-form, for example a gold ring-shaped preform, (not shown) is seated on the metallized rim 36A, and the ceramic device housing 22/gold pre-form/annular flange 60 subassembly is then subjected to a brazing process, as is well known to those skilled in the art related to brazing a ceramic material to a metallic flange. The brazing process melts the gold to thereby form a hermetic seal joining the flange 60 to the ceramic device housing 22 at the annular rim 36A.

The electrical energy power source 24 is a hermetically sealed electrochemical cell or capacitor comprising a metal or a ceramic casing 62. If ceramic, in a similar manner as with the ceramic material forming the device housing 22, the ceramic material forming the power source casing 62 is of an essentially high purity alumina. Whether metal or ceramic, the power source casing 62 has a surrounding sidewall extending from an annular proximal rim 62A to a closed distal end 62B. The annular proximal rim 62A is hermetically sealed to a metallic ferrule 64. The power source casing 62 is comprised of opposed upper and lower casing face walls 66 and 68 that extend to and meet with opposed curved casing edge walls 70 and 72. Together, the casing face walls 66, 68 and edge walls 70, 72 extend from the proximal rim 62A to the curved closed distal end 62B.

The ferrule 64 is of a biocompatible metal, for example titanium. If the casing 62 for the power source is metal, the ferrule 64 is attached to the proximal rim 62A of the casing 62 by a welding process, for example by laser welding. Alternatively, if the casing 62 is of a ceramic material, the ferrule 64 is hermetically secured to the proximal rim 62A of the ceramic casing by a brazing process, which is similar to the process that is used to braze the flange 60 to the proximal rim 36A of the device housing 22.

In any event, the ferrule 64 is sized and shaped to abut with the housing flange 60. A welding process, for example, a laser welding process is then used to connect the device housing 22 to the power source 24 at the flange 60 and ferrule 64. With the device housing 22 hermetically secured to the power source 24, the face walls 38, 40 and edge walls 42, 44 of the device housing 22 align with the face walls 66, 68 and edge walls 70, 72 of the power source casing 62. This provides the AIMD 12 comprising the device housing 22 secured to the power source 24 with a contoured exterior shape that is free of sharp edges and suitable to be implanted into a body tissue for an extended period of time without causing undue trauma to the surrounding body tissue.

While not shown in the drawings, the PCB 48 has opposite polarity terminal blocks that mate with opposite polarity terminal pins of the electrical energy power source 24. When the power source 24 is hermetically connected to the device housing 22 by welding the housing flange 60 to the power source ferrule 64, the power source is electrically connected to the PCB 48 and the electronic components supported on the PCB are electrically energized. Further, the power source 24 preferably has a third terminal pin (not shown) that is connected to the charging coil 50.

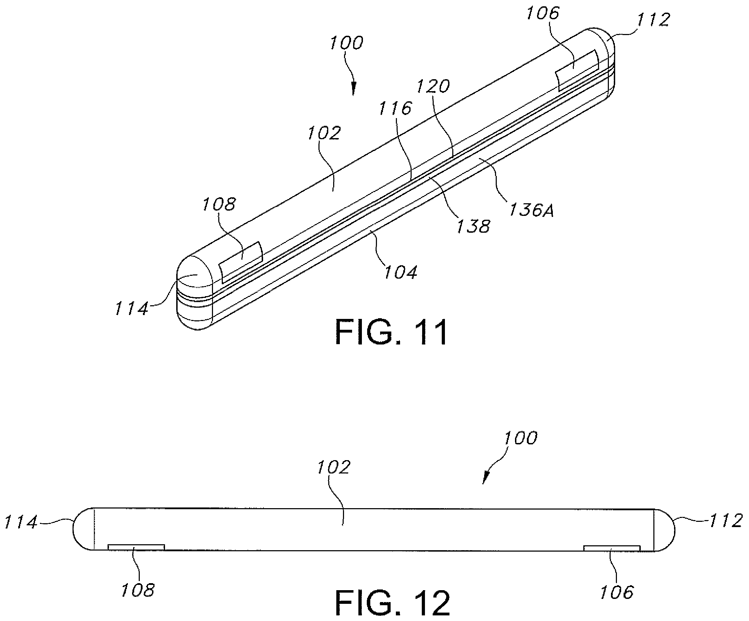

FIGs. 11 to 14 illustrate an alternate embodiment of a leadless AIMD 100 that is useful in the medical system 10 illustrated in FIG. 1. As with the previously described AIMD 12, the AIMD 100 comprises a non-conductive, elongate housing 102, for example of a ceramic material, connected to an electrical energy power source 104. The power source 104 can be a capacitor or a rechargeable battery, for example a hermetically sealed rechargeable Li-ion battery.

The AIMD 100 supports at least two spaced-apart discrete electrodes 106 and 108. Since they are not ring-shaped, the discrete electrodes 106 and 108 are configured to provide current to body tissue and/or to sense electrical signals from body tissue in a radiating pattern that is substantially directly in front of them. That is in comparison to the previously described ring-shaped electrodes 26 and 28, which provide current to body tissue and/or to sense electrical signals from body tissue in a 360° field about the ceramic housing. Moreover, while two electrodes 106, 108 are shown in this exemplary embodiment of an AIMD 100, that is the minimum number of electrodes that is needed for a functioning active implantable medical device. It is contemplated that there can three, four, and possibly more electrodes comprising the AIMD 100.

The device housing 102 is formed from a green-state ceramic material, for example essentially pure green-state alumina, having a U-shape in cross-section. The U-shaped housing sidewall 110 extends to opposed curved end walls 112 and 114. The sidewall 110 and end walls 112, 114 extend to a peripheral rim 116 that is hermetically connected to an annular flange 120 of a biocompatible material, for example titanium. The rim 118 of the ceramic housing 102 is connected to the flange 120 in a similar manner as previously described for connecting the proximal rim 36A of the device housing 22 for the AIMD 12 to the flange 60. That is, the peripheral rim 118 is provided with a metallization (not shown) comprising an adhesion layer of titanium and the wetting layer of either molybdenum or niobium. Then, a braze pre-form, for example a gold ring-shaped preform, (not shown) is seated on the metallized rim 118, and the ceramic device housing 102/gold pre-form/annular flange 120 subassembly is subjected to a brazing process that melts the gold to thereby form a hermetic seal joining the flange 120 to the ceramic device housing 102 at the peripheral rim 118.

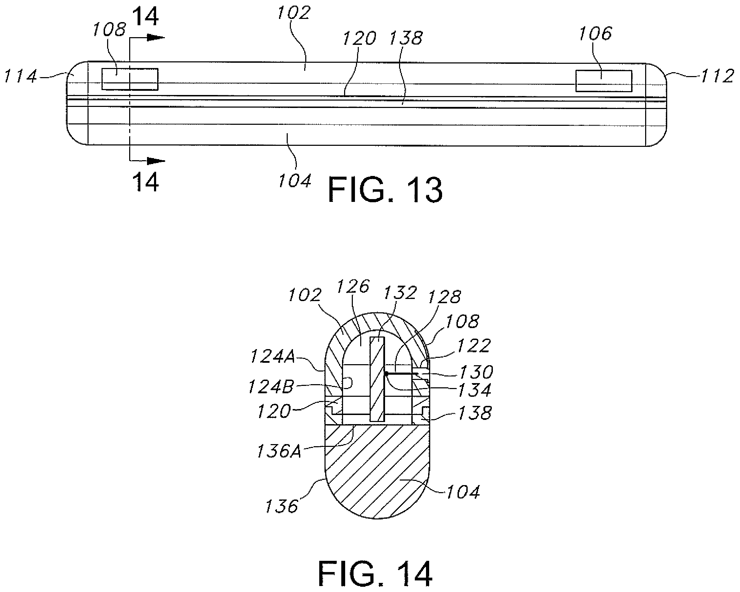

Prior to sintering, at least two vias 122 (only one via is shown in FIG. 14) are formed through the sidewall 110 of the green-state ceramic from an outer or body fluid side surface 124A to an inner or device side surface 124B (electronics side) defining an interior space 126 inside the alumina housing 102. Drilling is a preferred method for forming the vias 122, but they may also be formed by punching, laser drilling, water cutting, or any other equivalent process. After the vias 122 are formed, a pure platinum paste composition is injected under pressure or via vacuum into the vias. A connector pin 128 is inserted into the platinum paste so that the pin extends into the interior space 126 inside the device housing 102.

Referring now to the at least two discrete electrodes 106 and 108, they are of a biocompatible and electrically conductive material, preferably platinum, supported in matching shaped recesses in the body fluid side surface 124A of the green-state ceramic housing 102. The electrodes 106 and 108 are aligned and in direct contact with the body fluid ends of the platinum paste filled into a respective one of the at least two vias 122. The electrodes 106 and 108 are applied to the body fluid side surface 124A of the green-state ceramic housing 102 in a similar manner as the previously described electrodes 26, 28 are applied to the body fluid side surface 22A of the green-state ceramic housing 22 for the AIMD 12.

As an assembly, the green-state ceramic housing 102 supporting the discrete electrodes 106, 108 aligned and in direct contact with the platinum paste filled vias 122 is then subjected to a controlled co-firing or co-sintering process in ambient air that comprises a binder bakeout portion, a sintering portion, and a cool down portion. The sintering process results in a solid monolithic high purity alumina device housing 102 supporting the at least two electrodes 106 and 108 aligned and in direct physical and electrical contact with a body fluid side end of a respective one of the at least two conductive pathways 130 in the vias 122. The sintering process has transformed the platinum paste in the vias 122 into solid electrically conductive pathways 130 comprising platinum extending from the outer or body fluid side surface 124A of the housing where they directly electrically and physically contact the electrodes 106 and 108 to the inner surface 124B thereof defining the interior space 126 inside the device housing 102. A mutually conformal hermetic interface is thereby formed between the platinum conductive pathways 130 and the ceramic material of the device housing 102 defining the vias 122. The connector pin 128 extends outwardly from each of the conductive pathways 130 into the interior space 126 inside the device housing 102.

A printed circuit board (PCB) assembly 132 resides in the interior space 126 inside the device housing 102. The PCB 132 supports at least one, and preferably a plurality of electronic components (not shown) as an assembly that controls the various functions performed by the AIMD 100. As with the previously described AIMD 12, these include, but are not limited to, receiving sensed electrical signals pertaining to functions of the body tissue in which the AIMD 100 is implanted and for delivering electrical current pulses to the body tissue through the electrodes 106 and 108. The PCB 132 also supports a charging coil (not shown) connected to a charging circuit (not shown). The charging circuit is configured to convert RF or inductive energy received by the charging coil from the external charging pad 20 connected to the external charger 14 (FIG. 1) into a direct current voltage to charge the electrical power source 104 to power the electronic components of the PCB 132.

As shown in FIG. 14, with the PCB 132 residing in the interior space 126 inside the device housing 102, an electrical connection is made from the connector pins 128 extending outwardly from each of the electrically conductive pathways 130 by a re-flowed solder 134. The solder 134 connects the connector pins 128 to a respective electrical contact (not shown) supported on the PCB 132. The electrical contacts connect to electronic circuits of the PCB, and in turn, the electronic circuits draw power from the electrical power source 104 connected to the device housing 102.

In an alternate embodiment, the re-flowed solder 134 can be substituted with leaf springs (not shown) that are similar to the previously described leaf springs 56 and 58 and that bias into direct physical and electrical contact with the connector pins 128 extending outwardly from each of the conductive pathways 130 in turn electrically contacting the electrodes 106 and 108.

In a similar manner as the electrical energy power source 24 for the previously described AIMD 12, the electrical energy power source 104 is a hermetically sealed electrochemical cell or capacitor comprising a metal or a ceramic casing 136. Whether metal or ceramic, the power source casing 136 has a surrounding sidewall extending to an annular proximal rim 136A. The annular proximal rim 136A is hermetically sealed to a metallic ferrule 138. The ferrule 138 is of a biocompatible metal, for example titanium. If the casing 136 for the power source 104 is metal, the ferrule 138 is attached to the proximal rim 136A of the casing 136 by a welding process, for example, by laser welding. Alternatively, if the casing 136 is of a ceramic material, the ferrule 138 is hermetically secured to the proximal rim 136A of the ceramic casing by a brazing process, which is similar to the process that is used to braze the annular flange 120 to the peripheral rim 116 of the device housing 102.

A welding process, for example, a laser welding process is then used to connect the device housing 102 to the power source 104 at the flange 120 and ferrule 138. While not shown in the drawings, the PCB 132 has opposite polarity terminal blocks that mate with opposite polarity terminal pins of the electrical energy power source 104. When the power source 104 is hermetically connected to the device housing 102 by welding the housing flange 120 to the power source ferrule 138, the power source is electrically connected to the PCB 132 and the electronic components supported on the PCB are electrically energized. Further, the power source 104 preferably has a third terminal pin (not shown) that is connected to the charging coil.

The elongated shape of the leadless AIMD 12 shown in FIGs. 2 to 10 and the leadless AIMD 100 shown in FIGs. 11 to 14 allows the implantation procedure to be performed by injecting or inserting the AIMD into body tissue using of an insertion tool, typically through a very small incision. With the AIMD 12 or 100 implanted in body tissue, the electrical power source 24, 104 electrically connected to the PCB 48, 132 provides electrical power to the spaced-apart ring-shaped electrodes 26, 28 or discrete electrodes 106, 108. As ring-shaped members, the electrodes 26 and 28 are configured to electrically stimulate body tissue or sense biological signals in a 360° field about the device housing 22. Alternately, the discrete electrodes 106 and 108 shown in FIGs. 11 to 14 do not extend completely around a perimeter of the device housing. Instead, these electrodes are configured to stimulate body tissue or sense biological signals in a field that is centered directly in front of them.

It is noted that the elongated, annular shape of the ceramic device housing 22 for the leadless AIMD 12 means that the electrodes 26, 28 can be ring-shaped members that are continuously supported on the sidewall surrounding the longitudinal axis A-A of the ceramic housing 22. That is in contrast to the leadless AIMD 100 which does not have a ceramic housing with an annular shape. While the leadless AIMD 100 itself has an annular shape extending along a longitudinal axis, a portion of the AIMD 100 is provided by the electrical power source 104. For that reason, the electrode 106, 108 are discrete members that are not ring-shaped.

It is also within the scope of the present disclosure that an AIMD can have both ring-shaped electrodes and discrete electrodes. While at least two electrode are needed for a functioning device, there can be more than two electrodes of either a ring-shape or a discrete shape. In any event, the electrodes 26, 28 or 106, 108 are powered by the respective electrical power source 24, 104, or they can be powered through RF or inductive energy transmitted from the external charging pad 20 connected to the external charger 14 to the charging coil 50 electrically connected to the PCB 48, 132. The PCB supports electronic components that control the various functions of the medical device, including having the electrodes deliver electrical stimulation to a patient and sense biological signals from body tissue.

Thus, a method for powering an active implantable medical device (AIMD) comprises providing an active implantable medical device by providing a housing of a green-state alumina, the housing having a sidewall defining an open end and a sidewall thickness extending from a body fluid side surface to a device side surface. At least two vias are formed through the green-state housing sidewall thickness and the vias are then filled with an electrically conductive paste. At least two spaced-apart electrodes are supported on the green-state housing body fluid side surface, aligned with a respective one of the electrically conductive paste filled vias. The green-state alumina housing is then sintered to: solidify the alumina into the desired shape of a housing for the AIMD, transform the electrically conductive paste in the at least two vias into electrically conductive pathways extending from the device side surface of the housing to a respective one of the electrodes at the body fluid side surface, and secure the at least two electrodes to the body fluid side surface of the alumina housing. After sintering, a printed circuit board (PCB) assembly comprising a printed circuit board supporting at least one electronic component is moved through an open end and of the housing so that the PCB assembly resides inside the housing. The device side ends of each of the at least two platinum-containing pathways are then electrically connected to the at least one electronic component of the PCB assembly. Next, an electrical energy power source is secured to the open end of the housing so that the power source is electrically connected to the PCB assembly to provide electrical power to the at least one electronic component. Then, with the at least one electronic component being electrically energized, the at least two electrodes electrically connected to the PCB assembly by the respective platinum-containing pathways are configured to at least one of receive sensed electrical signals pertaining to functions of a body tissue in which the AIMD is implanted and deliver electrical current pulses to the body tissue.

It is appreciated that various modifications to the concepts described herein may be apparent to those skilled in the art without departing from the scope of the present invention as defined by the hereinafter appended claims.

## Claims

1. An active implantable medical device (AIMD) (12), comprising:
a) a non-conductive housing (22) having a housing sidewall extending from a proximal housing rim (36A) defining a housing opening to a closed distal end (36B), the proximal housing rim (36A) supporting a metallic annular flange (60), wherein the housing sidewall has a housing sidewall thickness extending from a body fluid side surface (22A) to a device side surface (22B) of the non-conductive housing (22);
b) at least two spaced-apart electrodes (26, 28) supported on the body fluid side surface (22A) of the non-conductive housing (22);
c) a printed circuit board (PCB) assembly (48) contained inside the housing (22) and comprising a printed circuit board supporting a plurality of electronic components;
d) at least two electrically conductive pathways (34) extending through the housing sidewall thickness from the body fluid side surface 22A to the device side surface (22B) of the non-conductive housing (22), wherein a body fluid side end of each of the at least two conductive pathways (34) is in electrical continuity with a respective one of the electrodes (26, 28), and wherein a device side end of each of the at least two conductive pathways (34) is in electrical continuity with the plurality of electronic components supported on the printed circuit board of the PCB assembly (48); and
e) an electrical power source (24) comprising a power source casing (62) having a surrounding sidewall extending from a proximal ring (62A) secured to a metallic ferrule (64), wherein the metallic ferrule (64) is secured to the metallic annular flange (60) to close the housing open end with the power source (24) being electrically connected to the PCB assembly (48) to provide electrical power to the plurality of electronic components,
f) wherein, with the plurality of electronic components being electrically energized, the at least two electrodes (26, 28) electrically connected to the PCB assembly (48) by the respective electrically conductive pathways (34) are configured to at least one of receive sensed electrical signals pertaining to functions of a body tissue and deliver electrical current pulses to the body tissue in which the AIMD (12) is implanted.

2. The AIMD of claim 1, wherein the electrically conductive pathways (34) are sintered conductive pathways, and preferably sintered platinum-containing conductive pathways.

3. The AIMD of claim 1 or claim 2, wherein a solder (52) or a leaf spring (58) electrically connects the PCB assembly (48) to the device side end of at least one of the at least two conductive pathways (34) in electrical continuity with a respective one of the at least two electrodes (26, 28).

4. The AIMD of any preceding claim, wherein the at least two electrodes (26, 28) are made of platinum.

5. The AIMD of any preceding claim, wherein each of the at least two electrodes (26, 28) is selected from a ring-shaped electrode (26, 28) and a discrete electrode (106, 108) that is not ring-shaped, and preferably wherein the at least two electrodes comprise (26, 28) at least one ring-shaped electrode (26, 28) and at least one discrete electrode that is not ring-shaped (106, 108).

6. The AIMD of any preceding claim, wherein the electrical power source (24) is a rechargeable electrical power source.

7. The AIMD of any preceding claim, further comprising a charging coil (50) connected to a charging circuit, wherein the charging circuit is configured to convert RF or inductive energy received from the charging coil (50) into a direct current voltage to charge the electrical power source (24) to power the PCB assembly (48).

8. The AIMD of any preceding claim, wherein the non-conductive housing (22) is made of alumina.

9. The AIMD of any preceding claim, wherein the electrical power source (24) is directly contactable by body fluids or body tissue.

10. The AIMD of any preceding claim, wherein the electrical power source (24) comprises a metallic casing (62) supporting the metallic ferrule (64) that is welded to the metallic annular flange (60) supported at the proximal housing rim (36A) of the non-conductive housing (22); or
wherein the electrical power source (24) comprises a ceramic casing (62) brazed to the metallic ferrule (64) that is welded to the metallic annular flange (60) supported at the proximal housing rim (36A) of the non-conductive housing (22).

11. A method for providing an active implantable medical device (AIMD) (12), comprising the steps of:
a) providing a housing (22) of a green-state alumina, the alumina housing (22) having a housing sidewall extending from a proximal housing rim (36A) defining an open end to a closed distal end (36B), wherein the housing sidewall has a sidewall thickness extending from a housing body fluid side surface (22A) to a housing device side surface (22B);
b) forming at least two vias (30) through the green-state alumina housing sidewall thickness;
c) filling the at least two vias (30) with a platinum-containing paste;
d) supporting at least two spaced-apart electrodes (26, 28) on the green-state alumina housing body fluid side surface (22A), wherein the electrodes (26, 28) are aligned with a respective one of the platinum-containing paste filled vias (30); and
e) sintering the green-state alumina housing (22) to:
i) solidify the alumina into a desired shape of a housing (22) for the AIMD,
ii) transform the platinum-containing paste in the at least two vias (30) into platinum-containing conductive pathways (34) extending from the device side surface (22B) of the alumina housing (22) to a respective one of the electrodes (26, 28) at the body fluid side surface (22A), and
iii) secure the at least two electrodes (26, 28) to the body fluid side surface (22A) of the alumina housing (22);
f) brazing a metallic annular flange (60) to the proximal housing rim (36A) at the open end of the alumina housing (22);
g) moving a printed circuit board (PCB) assembly (48) comprising a printed circuit board supporting a plurality of electronic components through the alumina housing (22) open end so that the PCB assembly (48) resides inside the alumina housing (22) followed by connecting the PCT assembly (48) to the at least two platinum-containing pathways (34) so that the at least two platinum-containing pathways (34) are in electrical continuity with the plurality of electronic components of the PCB assembly (48);
h) securing an electrical power source (24) to the metallic annular flange (60), wherein the electrical power source (24) comprises a power source casing (62) having a surrounding sidewall extending from a proximal ring (62A) secured to a metallic ferrule (64) and the electrical power source (24) is secured to the metallic annular flange (60) via the metallic ferrule (64) to close the open end of the alumina housing (22) with the power source (24) being electrically connected to the PCB assembly (48) to provide electrical power to the plurality of electronic components of the PCB assembly (48),
i) wherein, with the plurality of electronic components being electrically energized, the at least two electrodes (26, 28) electrically connected to the PCB assembly (48) by the respective platinum-containing pathways (34) are configured to at least one of receive sensed electrical signals pertaining to functions of a body tissue and deliver electrical current pulses to the body tissue in which the AIMD (12) is implanted.

12. The method of claim 11, including electrically connecting the PCB assembly (48) to the device side end of at least one of the at least two platinum-containing conductive pathways (34) in electrical continuity with a respective one of the at least two electrodes (26, 28) with a solder (52) or a leaf spring (56).

13. The method of claim 11 or claim 12, including:
a) providing each of the at least two electrodes (26, 28) as a ring-shaped electrode (26, 28) or a discrete electrode that is not ring-shaped (106, 108); and/or
b) providing the at least two electrodes (26, 28) as platinum electrodes and the electrical power source (24) as a rechargeable electrical energy power source.

14. The method of any of claims 11 to 13, including providing the power source (24) being directly contactable by body fluids or body tissue.

15. The method of any of claims 11 to 14, including providing the electrical power source (24) comprising a metallic casing (62) supporting the metallic ferrule (64), and welding the metallic ferrule (64) to the metallic annular flange (60) supported at the proximal housing rim (36A) of the alumina housing (22); or
providing the electrical power source (24) comprising a ceramic casing (62) supporting a brazed metallic ferrule (64), and welding the metallic ferrule (64) to the metallic annular flange (60) supported at the proximal housing rim (36A) of the alumina housing (22).

## Patentansprüche

1. Aktive implantierbare medizinische Vorrichtung (AIMD) (12), umfassend:
a) ein nicht-leitfähiges Gehäuse (22), aufweisend eine Gehäuseseitenwand, die sich von einem proximalen Gehäuserand (36A), der eine Gehäuseöffnung definiert, zu einem geschlossenen distalen Ende (36B) erstreckt, wobei der proximale Gehäuserand (36A) einen metallischen kranzförmigen Flansch (60) trägt, wobei die Gehäuseseitenwand eine Gehäuseseitenwanddicke aufweist, die sich von einer Körperflüssigkeitsseitenfläche (22A) zu einer Vorrichtungsseitenfläche (22B) des nicht-leitfähigen Gehäuses (22) erstreckt;
b) mindestens zwei voneinander beabstandete Elektroden (26, 28), die auf der Körperflüssigkeitsseitenfläche (22A) des nicht-leitfähigen Gehäuses (22) getragen werden;
c) eine Leiterplattenanordnung (PCB-Anordnung) (48), die im Inneren des Gehäuses (22) enthalten ist und eine Leiterplatte umfasst, die eine Vielzahl von elektronischen Komponenten trägt;
d) mindestens zwei elektrisch leitfähige Bahnen (34), die sich durch die Gehäuseseitenwanddicke von der Körperflüssigkeitsseitenfläche 22A zu der Vorrichtungsseitenfläche (22B) des nicht-leitfähigen Gehäuses (22) erstrecken, wobei ein Körperflüssigkeitsseitenende jeder der mindestens zwei leitfähigen Bahnen (34) in elektrischer Kontinuität mit einer jeweiligen der Elektroden (26, 28) ist, und wobei ein Vorrichtungsseitenende jeder der mindestens zwei leitfähigen Bahnen (34) in elektrischer Kontinuität mit der Vielzahl von elektronischen Komponenten ist, die auf der Leiterplatte der PCB-Anordnung (48) getragen wird; und
e) eine elektrische Leistungsquelle (24), umfassend ein Leistungsquellenverkleidung (62), aufweisend eine umschließende Seitenwand, die sich von einem proximalen Ring (62A) erstreckt, der an einer metallischen Muffe (64) gesichert ist, wobei die metallische Muffe (64) an dem metallischen kranzförmigen Flansch (60) gesichert ist, um das offene Gehäuseende zu schließen, wobei die Leistungsquelle (24) mit der PCB-Anordnung (48) elektrisch verbunden ist, um elektrische Leistung an die Vielzahl von elektronischen Komponenten bereitzustellen,
f) wobei, wenn die Vielzahl von elektronischen Komponenten mit elektrischer Energie versorgt wird, die mindestens zwei Elektroden (26, 28), die durch die jeweiligen elektrisch leitfähigen Bahnen (34) mit der PCB-Anordnung (48) elektrisch verbunden sind, zu mindestens einem von einem Empfangen von erfassten elektrischen Signalen in Bezug auf Funktionen eines Körpergewebes und einem Abgeben von elektrischen Stromimpulsen an das Körpergewebe, in dem die AIMD (12) implantiert ist, konfiguriert sind.

2. AIMD nach Anspruch 1, wobei die elektrisch leitfähigen Bahnen (34) gesinterte leitfähige Bahnen sind und vorzugsweise gesinterte platinhaltige leitfähige Bahnen sind.

3. AIMD nach Anspruch 1 oder Anspruch 2, wobei ein Lötmittel (52) oder eine Blattfeder (58) die PCB-Anordnung (48) mit dem Vorrichtungsseitenende mindestens einer der mindestens zwei leitfähigen Bahnen (34) in elektrischer Kontinuität mit einer jeweiligen der mindestens zwei Elektroden (26, 28) elektrisch verbindet.

4. AIMD nach einem vorhergehenden Anspruch, wobei die mindestens zwei Elektroden (26, 28) aus Platin hergestellt sind.

5. AIMD nach einem vorhergehenden Anspruch, wobei jede der mindestens zwei Elektroden (26, 28) aus einer ringförmigen Elektrode (26, 28) und einer separaten Elektrode (106, 108), die nicht ringförmig ist, ausgewählt ist, und vorzugsweise wobei die mindestens zwei Elektroden (26, 28) mindestens eine ringförmige Elektrode (26, 28) und mindestens eine separate Elektrode, die nicht ringförmig ist (106, 108), umfassen.

6. AIMD nach einem vorhergehenden Anspruch, wobei die elektrische Leistungsquelle (24) eine wiederaufladbare elektrische Leistungsquelle ist.

7. AIMD nach einem vorhergehenden Anspruch, ferner umfassend eine Ladespule (50) die mit einem Ladestromkreis verbunden ist, wobei der Ladestromkreis dazu konfiguriert ist, HF- oder induktive Energie, die von der Ladespule (50) empfangen wird, in eine Gleichstromspannung umzuwandeln, um die elektrische Leistungsquelle (24) zu laden, um die PCB-Anordnung (48) anzutreiben.

8. AIMD nach einem vorhergehenden Anspruch, wobei das nicht-leitfähige Gehäuse (22) aus Aluminiumoxid hergestellt ist.

9. AIMD nach einem vorhergehenden Anspruch, wobei die elektrische Leistungsquelle (24) durch Körperflüssigkeiten oder Körpergewebe direkt berührt werden kann.

10. AIMD nach einem vorhergehenden Anspruch, wobei die elektrische Leistungsquelle (24) eine metallische Verkleidung (62) umfasst, die die metallische Muffe (64) trägt, die an den metallischen kranzförmigen Flansch (60) geschweißt ist, der an dem proximalen Gehäuserand (36A) des nicht-leitfähigen Gehäuses (22) getragen wird; oder
wobei die elektrische Leistungsquelle (24) eine keramische Verkleidung (62) umfasst, die an die metallische Muffe (64) gelötet ist, die an den metallischen kranzförmigen Flansch (60) geschweißt ist, der an dem proximalen Gehäuserand (36A) des nicht-leitfähigen Gehäuses (22) getragen wird.

11. Verfahren zur Bereitstellung einer aktiven implantierbaren medizinischen Vorrichtung (AIMD) (12), umfassend die Schritte:
a) Bereitstellen eines Gehäuses (22) aus einem Aluminiumoxid im Grünzustand, wobei das Aluminiumoxid-Gehäuse (22) eine Gehäuseseitenwand aufweist, die sich von einem proximalen Gehäuserand (36A), der ein offenes Ende definiert, zu einem geschlossenen distalen Ende (36B) erstreckt, wobei die Gehäuseseitenwand eine Seitenwanddicke aufweist, die sich von einer Körperflüssigkeitsseitenfläche (22A) zu einer Vorrichtungsseitenfläche (22B) erstreckt;
b) Formen von mindestens zwei Durchkontaktierungen (30) durch die Seitenwanddicke des Gehäuses aus Aluminiumoxid im Grünzustand;
c) Füllen der mindestens zwei Durchkontaktierungen (30) mit einer platinhaltigen Paste;
d) Tragen von mindestens zwei voneinander beabstandeten Elektroden (26, 28) auf der Körperflüssigkeitsseitenfläche (22A) des Gehäuses aus Aluminiumoxid im Grünzustand, wobei die Elektroden (26, 28) auf eine jeweilige der mit platinhaltiger Paste gefüllten Durchkontaktierungen (30) ausgerichtet sind; und
e) Sintern des Gehäuses (22) aus Aluminiumoxid im Grünzustand zum:
(i) Verfestigen des Aluminiumoxids zu einer gewünschten Form eines Gehäuses (22) für die AIMD,
(ii) Umwandeln der platinhaltigen Paste in den mindestens zwei Durchkontaktierungen (30) in platinhaltige leitfähige Bahnen (34), die sich von der Vorrichtungsseitenfläche (22B) des Aluminiumoxid-Gehäuses (22) zu einer jeweiligen der Elektroden (26, 28) an der Körperflüssigkeitsseitenfläche (22A) erstrecken, und
(iii) Sichern der mindestens zwei Elektroden (26, 28) an der Körperflüssigkeitsseitenfläche (22A) des Aluminiumoxid-Gehäuses (22);
f) Löten eines metallischen kranzförmigen Flanschs (60) an den proximalen Gehäuserand (36A) an dem offenen Ende des Aluminiumoxid-Gehäuses (22);
g) Bewegen einer Leiterplattenanordnung (PCB-Anordnung) (48), umfassend eine Leiterplatte, die eine Vielzahl von elektronischen Komponenten trägt, durch das offene Ende des Aluminiumoxid-Gehäuses (22), so dass sich die PCB-Anordnung (48) im Inneren des Aluminiumoxid-Gehäuses (22) befindet, gefolgt von einem Verbinden der PCT-Anordnung (48) mit den mindestens zwei platinhaltigen Bahnen (34), so dass die mindestens zwei platinhaltigen Bahnen (34) in elektrischer Kontinuität mit der Vielzahl von elektronischen Komponenten der PCB-Anordnung (48) sind;
h) Sichern einer elektrischen Leistungsquelle (24) an dem metallischen kranzförmigen Flansch (60), wobei die elektrische Leistungsquelle (24) ein Leistungsquellenverkleidung (62) umfasst, aufweisend eine umschließende Seitenwand, die sich von einem proximalen Ring (62A) erstreckt, der an einer metallischen Muffe (64) gesichert ist, und die elektrische Leistungsquelle (24) mittels der metallischen Muffe (64) an dem metallischen kranzförmigen Flansch (60) gesichert ist, um das offene Ende des Aluminiumoxid-Gehäuses (22) zu schließen, wobei die Leistungsquelle (24) mit der PCB-Anordnung (48) elektrisch verbunden ist, um elektrische Leistung an die Vielzahl von elektronischen Komponenten der PCB-Anordnung (48) bereitzustellen,
i) wobei, wenn die Vielzahl von elektronischen Komponenten mit elektrischer Energie versorgt wird, die mindestens zwei Elektroden (26, 28), die durch die jeweiligen platinhaltigen Bahnen (34) mit der PCB-Anordnung (48) elektrisch verbunden sind, zu mindestens einem von einem Empfangen von erfassten elektrischen Signalen in Bezug auf Funktionen eines Körpergewebes und einem Abgeben von elektrischen Stromimpulsen an das Körpergewebe, in dem die AIMD (12) implantiert ist, konfiguriert sind.

12. Verfahren nach Anspruch 11, einschließend ein elektrisches Verbinden der PCB-Anordnung (48) mit dem Vorrichtungsseitenende mindestens einer der mindestens zwei platinhaltigen leitfähigen Bahnen (34) in elektrischer Kontinuität mit einer jeweiligen der mindestens zwei Elektroden (26, 28) mit einem Lötmittel (52) oder einer Blattfeder (56).

13. Verfahren nach Anspruch 11 oder Anspruch 12, einschließend:
a) Bereitstellen jeder der mindestens zwei Elektroden (26, 28) als eine ringförmige Elektrode (26, 28) oder eine separate Elektrode, die nicht ringförmig ist (106, 108); und/oder
b) Bereitstellen der mindestens zwei Elektroden (26, 28) als Platin-Elektroden und der elektrischen Leistungsquelle (24) als eine wiederaufladbare elektrische Energieleistungsquelle.

14. Verfahren nach einem der Ansprüche 11 bis 13, einschließend ein Bereitstellen der Leistungsquelle (24), die durch Körperflüssigkeiten oder Körpergewebe direkt berührt werden kann.

15. Verfahren nach einem der Ansprüche 11 bis 14, einschließend ein Bereitstellen der elektrischen Leistungsquelle (24), umfassend eine metallische Verkleidung (62), die die metallische Muffe (64) trägt, und ein Schweißen der metallischen Muffe (64) an den metallischen kranzförmigen Flansch (60), der an dem proximalen Gehäuserand (36A) des Aluminiumoxid-Gehäuses (22) getragen wird; oder
ein Bereitstellen der elektrischen Leistungsquelle (24), umfassend eine keramische Verkleidung (62), die an eine gelötete metallische Muffe (64) trägt, und ein Schweißen der metallischen Muffe (64) an den metallischen kranzförmigen Flansch (60), der an dem proximalen Gehäuserand (36A) des Aluminiumoxid-Gehäuses (22) getragen wird.

## Revendications

1. Dispositif médical implantable actif (AIMD) (12), comprenant :
a) un boîtier non conducteur (22) ayant une paroi latérale de boîtier s'étendant depuis un rebord de boîtier proximal (36A) délimitant une ouverture de boîtier jusqu'à une extrémité distale fermée (36B), le rebord de boîtier proximal (36A) portant une bride annulaire métallique (60), la paroi latérale de boîtier ayant une épaisseur de paroi latérale de boîtier s'étendant depuis une surface latérale côté fluide corporel (22A) jusqu'à une surface latérale côté dispositif (22B) du boîtier non conducteur (22) ;
b) au moins deux électrodes espacées (26, 28) supportées sur la surface latérale côté fluide corporel (22A) du boîtier non conducteur (22) ;
c) un ensemble carte de circuit imprimé (PCB) (48) contenu à l'intérieur du boîtier (22) et comprenant une carte de circuit imprimé portant une pluralité de composants électroniques ;
d) au moins deux voies conductrices d'électricité (34) s'étendant à travers l'épaisseur de paroi latérale de boîtier depuis la surface latérale côté fluide corporel 22A jusqu'à la surface latérale côté dispositif (22B) du boîtier non conducteur (22), une extrémité côté fluide corporel de chacune des au moins deux voies conductrices (34) étant en continuité électrique avec l'une respective des électrodes (26, 28), et une extrémité côté dispositif de chacune des au moins deux voies conductrices (34) étant en continuité électrique avec la pluralité de composants électroniques supportée sur la carte de circuit imprimé de l'ensemble PCB (48) ; et
e) une source d'énergie électrique (24) comprenant un logement de source d'énergie (62) ayant une paroi latérale périphérique s'étendant depuis un anneau proximal (62A) fixé à une virole métallique (64), la virole métallique (64) étant fixée à la bride annulaire métallique (60) pour fermer l'extrémité ouverte du boîtier, la source d'énergie (24) étant connectée électriquement à l'ensemble PCB (48) pour fournir de l'énergie électrique à la pluralité de composants électroniques,
f) dans lequel, la pluralité de composants électroniques étant alimentée électriquement, les au moins deux électrodes (26, 28) connectées électriquement à l'ensemble PCB (48) par les voies conductrices d'électricité respectives (34) sont configurées pour au moins l'un de la réception de signaux électriques détectés relatifs à des fonctions d'un tissu corporel et l'apport d'impulsions de courant électrique au tissu corporel dans lequel l'AIMD (12) est implanté.

2. AIMD selon la revendication 1, dans lequel les voies conductrices d'électricité (34) sont des voies conductrices frittées, et préférablement des voies conductrices frittées contenant du platine.

3. AIMD selon la revendication 1 ou la revendication 2, dans lequel une soudure (52) ou un ressort à lames (58) connecte électriquement l'ensemble PCB (48) à l'extrémité côté dispositif d'au moins une des au moins deux voies conductrices (34) en continuité électrique avec l'une respective des au moins deux électrodes (26, 28).

4. AIMD selon l'une quelconque des revendications précédentes, dans lequel les au moins deux électrodes (26, 28) sont en platine.

5. AIMD selon l'une quelconque des revendications précédentes, dans lequel chacune des au moins deux électrodes (26, 28) est sélectionnée parmi une électrode en forme d'anneau (26, 28) et une électrode distincte (106, 108) qui n'est pas en forme d'anneau, et préférablement dans lequel les au moins deux électrodes (26, 28) comprennent au moins une électrode en forme d'anneau (26, 28) et au moins une électrode distincte qui n'est pas en forme d'anneau (106, 108).

6. AIMD selon l'une quelconque des revendications précédentes, dans lequel la source d'énergie électrique (24) est une source d'énergie électrique rechargeable.

7. AIMD selon l'une quelconque des revendications précédentes, comprenant en outre une bobine de charge (50) connectée à un circuit de charge, le circuit de charge étant configuré pour convertir de l'énergie RF ou inductive reçue de la bobine de charge (50) en une tension de courant continu pour charger la source d'énergie électrique (24) afin d'alimenter l'ensemble PCB (48).

8. AIMD selon l'une quelconque des revendications précédentes, dans lequel le boîtier non conducteur (22) est en alumine.

9. AIMD selon l'une quelconque des revendications précédentes, dans lequel la source d'énergie électrique (24) peut être directement mise en contact avec des fluides corporels ou des tissus corporels.

10. AIMD selon l'une quelconque des revendications précédentes, dans lequel la source d'énergie électrique (24) comprend un logement métallique (62) portant la virole métallique (64) qui est soudée à la bride annulaire métallique (60) supportée au niveau du rebord de boîtier proximal (36A) du boîtier non conducteur (22) ; ou
dans lequel la source d'énergie électrique (24) comprend un logement en céramique (62) brasé sur la virole métallique (64) qui est soudée à la bride annulaire métallique (60) supportée au niveau du rebord de boîtier proximal (36A) du boîtier non conducteur (22).

11. Procédé de production d'un dispositif médical implantable actif (AIMD) (12), comprenant les étapes qui consistent à :
a) fournir un boîtier (22) en alumine à l'état vert, le boîtier en alumine (22) ayant une paroi latérale de boîtier s'étendant depuis un rebord de boîtier proximal (36A) délimitant une extrémité ouverte jusqu'à une extrémité distale fermée (36B), la paroi latérale de boîtier ayant une épaisseur de paroi latérale s'étendant depuis une surface latérale côté fluide corporel de boîtier (22A) jusqu'à une surface latérale côté dispositif de boîtier (22B) ;
b) former au moins deux trous d'interconnexion (30) à travers l'épaisseur de la paroi latérale du boîtier en alumine à l'état vert ;
c) remplir les au moins deux trous d'interconnexion (30) avec une pâte contenant du platine ;
d) monter au moins deux électrodes espacées (26, 28) sur la surface latérale côté fluide corporel du boîtier en alumine à l'état vert (22A), les électrodes (26, 28) étant alignées avec l'un respectif des trous d'interconnexion remplis de pâte contenant du platine (30) ; et
e) fritter le boîtier en alumine à l'état vert (22) pour :
(i) solidifier l'alumine en lui donnant une forme souhaitée d'un boîtier (22) pour l'AIMD,
(ii) transformer la pâte contenant du platine dans les au moins deux trous d'interconnexion (30) en des voies conductrices contenant du platine (34) s'étendant depuis la surface latérale côté dispositif (22B) du boîtier en alumine (22) jusqu'à l'une respective des électrodes (26, 28) au niveau de la surface latérale côté fluide corporel (22A), et
(iii) fixer les au moins deux électrodes (26, 28) à la surface latérale côté fluide corporel (22A) du boîtier en alumine (22) ;
f) braser une bride annulaire métallique (60) sur le rebord de boîtier proximal (36A) à l'extrémité ouverte du boîtier en alumine (22) ;
g) déplacer un ensemble carte de circuit imprimé (PCB) (48) comprenant une carte de circuit imprimé portant une pluralité de composants électroniques à travers l'extrémité ouverte du boîtier en alumine (22) de telle sorte que l'ensemble PCB (48) réside à l'intérieur du boîtier en alumine (22), et ensuite connecter l'ensemble PCT(48) aux au moins deux voies contenant du platine (34) de telle sorte que les au moins deux voies contenant du platine (34) soient en continuité électrique avec la pluralité de composants électroniques de l'ensemble PCB (48) ;
h) fixer une source d'énergie électrique (24) à la bride annulaire métallique (60), la source d'énergie électrique (24) comprenant un logement de source d'énergie (62) ayant une paroi latérale périphérique s'étendant depuis un anneau proximal (62A) fixé à une virole métallique (64) et la source d'énergie électrique (24) étant fixée à la bride annulaire métallique (60) par l'intermédiaire de la virole métallique (64) pour fermer l'extrémité ouverte du boîtier en alumine (22), la source d'énergie (24) étant connectée électriquement à l'ensemble PCB (48) pour fournir de l'énergie électrique à la pluralité de composants électroniques de l'ensemble PCB (48),
i) dans lequel, la pluralité de composants électroniques étant alimentée électriquement, les au moins deux électrodes (26, 28) connectées électriquement à l'ensemble PCB (48) par les voies contenant du platine respectives (34) sont configurées pour au moins l'un de la réception de signaux électriques détectés relatifs à des fonctions d'un tissu corporel et l'apport d'impulsions de courant électrique au tissu corporel dans lequel l'AIMD (12) est implanté.

12. Procédé selon la revendication 11, comprenant une connexion électrique de l'ensemble PCB (48) à l'extrémité côté dispositif d'au moins une des au moins deux voies conductrices contenant du platine (34) en continuité électrique avec l'une respective des au moins deux électrodes (26, 28) par une soudure (52) ou un ressort à lames (56).

13. Procédé selon la revendication 11 ou la revendication 12, comprenant :
a) la fourniture de chacune des au moins deux électrodes (26, 28) sous la forme d'une électrode en forme d'anneau (26, 28) ou d'une électrode distincte qui n'est pas en forme d'anneau (106, 108) ; et/ou
b) la fourniture des au moins deux électrodes (26, 28) sous forme d'électrodes en platine et de la source d'énergie électrique (24) sous forme de source d'énergie électrique rechargeable.

14. Procédé selon l'une quelconque des revendications 11 à 13, comprenant une fourniture de la source d'énergie (24) pouvant être directement mise en contact avec des fluides corporels ou des tissus corporels.

15. Procédé selon l'une quelconque des revendications 11 à 14, comprenant une fourniture de la source d'énergie électrique (24) comprenant un logement métallique (62) portant la virole métallique (64), et un soudage de la virole métallique (64) à la bride annulaire métallique (60) supportée au niveau du rebord de boîtier proximal (36A) du boîtier en alumine (22) ; ou
une fourniture de la source d'énergie électrique (24) comprenant un logement en céramique (62) portant une virole métallique brasée (64), et un soudage de la virole métallique (64) à la bride annulaire métallique (60) supportée au niveau du rebord de boîtier proximal (36A) du boîtier en alumine (22).
